# EUROPEAN PATENT APPLICATION

(11) **EP 2 915 510 A1**
(43) Date of publication of application: **09.09.2015**
(21) Application number: 14169746.6
(22) Date of filing: 23.05.2014
(51) Int. Cl.: A61F 5/37, A61B 19/00

(54) **Armrest apparatus**

(30) Priority: 05.03.2014 US 201414198572
(71) Applicant: Chen, Michael, Baldwin Park, CA 91706 (US); Wei, Wen, Baldwin Park, CA 91706 (US); Chen, Billy, Baldwin Park, CA 91706 (US)
(72) Inventor: Miller, Adam, California, 90026 (US); Chen, Michael, California, 91706 (US)
(74) Representative: Jennings, Nigel Robin

(57) **Abstract**

An armrest apparatus (10) includes a first support post (20) having an open top end (28) and a bottom end (22). A second support post (30) has a top end (38) and a bottom end (32). The top end (38) of the second support post (30) is pivotally fixed with the top end of the first support post (20) between an extended position and a collapsed position. The bottom end of each support post (20, 30) includes two opposing legs (80), each of which are fixed at a proximal end (82) thereof with the bottom end of the support post respectively. Each leg (82) is pivotal between an extended configuration and a collapsed configuration. An armrest support post (40) is telescopingly fixed with the open top end of the first post (20) at a lower end thereof. The armrest support post (40) is fixed with an armrest adjustment mechanism (120) at an upper end (48) thereof that is fixed with a cushioned armrest (50).

## Description

### FIELD OF THE INVENTION

This invention relates to supports, and more particularly to an adjustable armrest apparatus.

### DISCUSSION OF RELATED ART

When performing manual work with the hands for extended periods of time, a person's arms can easily become fatigued. If the manual work is performed on a desk or table, typically a person will rest his arms on the table while performing the work. However, in some situations a desk or table is not practical or available, such as for tattoo artists for example. Often the surface upon which the person works is held at an irregular angle and location, requiring the person to hold his arms steady without convenient arm support.

Several arm-rest devices are known in the prior art for reducing fatigue caused by holding one's arms in an irregular or unusual position for an extended time. For example, US 1,025,476 to Mellen on May 7, 1912, teaches an adjustable arm rest device for supporting a person's arm while being fitted for a glove. Such a device is not well suited for allowing a person to rest his arms while his arms are in a straight position, but rather requires the arm to be bent at the elbow. Further, such a device does not allow for an angular positioning of the armrest in both tilt and roll directions.

The portable massage chair, disclosed in US 2005/0035644 to Lloyd on Feb. 17, 2005, includes an armrest portion that is angularly adjustable in a tilt direction, but not a roll direction. Such a device is collapsible, but is bulky and too complicated to be used in many applications other than its intended use.

US 2011/0266411 to Silverbert et al. on Nov. 3, 2011 teaches a movable arm support system that allows for a wide variety of arm positions at a fixed height above a ground surface to which the device is bolted. Such a device is not portable or well-suited for use at other than a particular fixed height above the ground. Further, such a device does not collapse for convenient storage or transport.

US 2,630,288 to Eubanks, Sr., on March 3, 1953 teaches an adjustable arm and leg rest. Such a device is adjustable in tilt and yaw directions, but not roll. Further, such a device is not easily collapsed for convenient storage or transport.

Therefore, there is a need for an armrest device that may be positioned to support a user's arms at varying heights above a floor surface, and at various angle in the directions of tilt, roll and yaw. Such a needed device would be collapsible into a compact configuration for easy storage and transport, and would further be relatively inexpensive to manufacture and easy to use. The present invention accomplishes these objectives.

### SUMMARY OF THE INVENTION

The present device is an armrest apparatus for resting on a horizontal surface and for supporting the arm or arms of a user. A first support post has an open top end and a bottom end. A second support post has a top end and a bottom end. The top end of the second support post is pivotally fixed with the top end of the first support post between an extended position and a collapsed position.

The bottom end of each support post includes two opposing legs, each of which are fixed at a proximal end thereof with the bottom end of the support post respectively. Each leg is pivotal between an extended configuration and a collapsed configuration. A collapsible angle brace is fixed between the post and a distal end of the leg.
An armrest support post is telescopingly fixed with the open top end of the first post at a lower end thereof. The armrest support post is fixed with an armrest adjustment mechanism at an upper end thereof. Preferably the armrest support post includes a plurality of adjustment apertures along one side thereof, and the second support post is pivotally fixed with the first support post at a first pivot mechanism. An adjustment knob traversing the first pivot mechanism and the first support post engages one of the adjustment apertures of the armrest support post to selectively fix the first support post and the armrest support post mutually together.

A cushioned armrest is fixed with the armrest adjustment mechanism, preferably with a ball joint for allowing the armrest to be adjusted angularly with respect to the armrest support post.

In one embodiment, a forward leg projects forwardly from the bottom end of the support post and two legs projecting away from the support post generally rearwardly and separated by an angle of less than 180-degrees together support the armrest apparatus on the horizontal surface, precluding the need for the second support post. In use, the first and second support posts may be separated into the extended position, each leg placed in its extended configuration for supporting the armrest on the horizontal surface. The height of the armrest support post may be adjusted within the first support post, and the angle of the armrest may be adjusted with the armrest adjustment mechanism. The armrest apparatus may be placed in a position suited for supporting the arm or arms of a person (not shown).

The present invention is an armrest device that may be positioned to support a user's arms at varying heights above a floor surface, and at various angle in the directions of tilt, roll and yaw. The present device is collapsible into a compact configuration for easy storage and transport, and is relatively inexpensive to manufacture and easy to use. Other features and advantages of the present invention will become apparent from the following more detailed description, taken in conjunction with the accompanying drawings, which illustrate, by way of example, the principles of the invention.

### DESCRIPTION OF THE DRAWINGS

FIG. 1 is a perspective view of the invention, illustrating an armrest apparatus in a fully extended configuration;
FIG. 2 is a side elevational view, partially broken away, of FIG. 1;
FIG. 3 is a perspective view of the invention, illustrated in a collapsed configuration;
FIG. 4 is a side perspective view of FIG. 3;
FIG. 5 is a cross-sectional view of a ball joint of the invention, taken generally along lines 5-5 of FIG. 2;
FIG. 6 is a perspective view of an alternate embodiment of the invention; and
FIG. 7 is a right-side elevational view of the embodiment of FIG. 6.

### DETAILED DESCRIPTION OF THE PREFERRED EMBODIMENT

Illustrative embodiments of the invention are described below. The following explanation provides specific details for a thorough understanding of and enabling description for these embodiments. One skilled in the art will understand that the invention may be practiced without such details. In other instances, well-known structures and functions have not been shown or described in detail to avoid unnecessarily obscuring the description of the embodiments.

Unless the context clearly requires otherwise, throughout the description and the claims, the words "comprise," "comprising," and the like are to be construed in an inclusive sense as opposed to an exclusive or exhaustive sense; that is to say, in the sense of "including, but not limited to." Words using the singular or plural number also include the plural or singular number respectively. Additionally, the words "herein," "above," "below" and words of similar import, when used in this application, shall refer to this application as a whole and not to any particular portions of this application. When the claims use the word "or" in reference to a list of two or more items, that word covers all of the following interpretations of the word: any of the items in the list, all of the items in the list and any combination of the items in the list. When the word "each" is used to refer to an element that was previously introduced as being at least one in number, the word "each" does not necessarily imply a plurality of the elements, but can also mean a singular element.

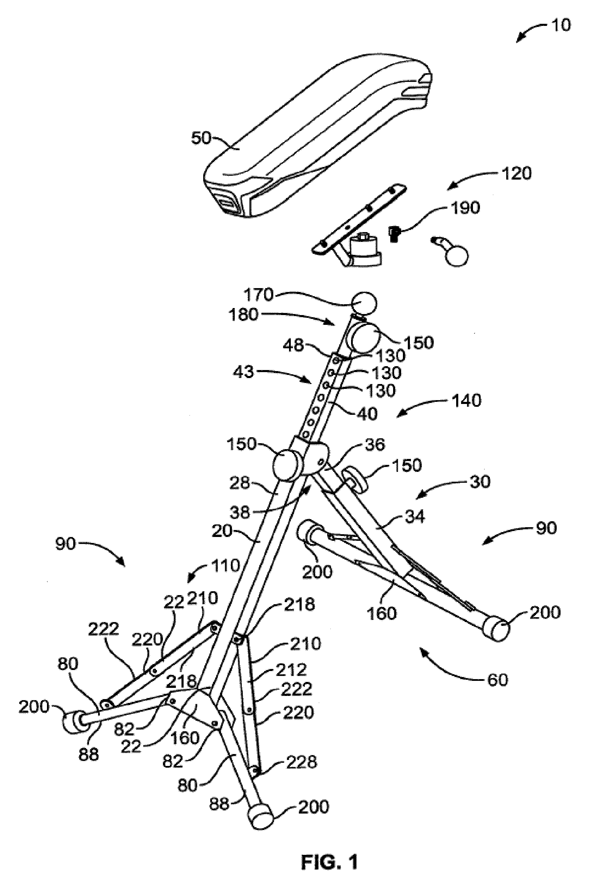

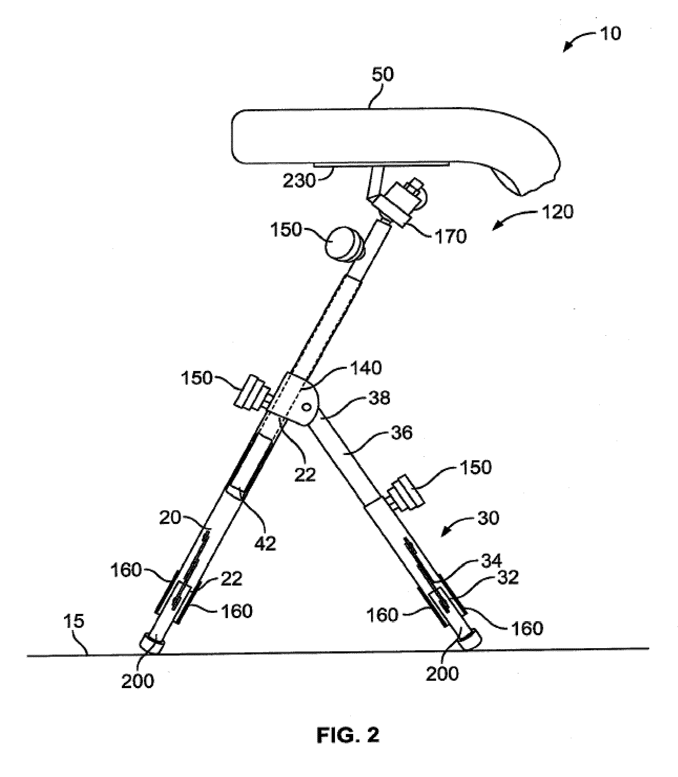

FIGS. 1 and 2 illustrate an armrest apparatus 10 for resting on a horizontal surface 15 and for supporting the arm or arms of a user (not shown). A first support post 20 has an open top end 28 and a bottom end 22. Preferably the first support post 20 is made from a rigid metallic, steel material.

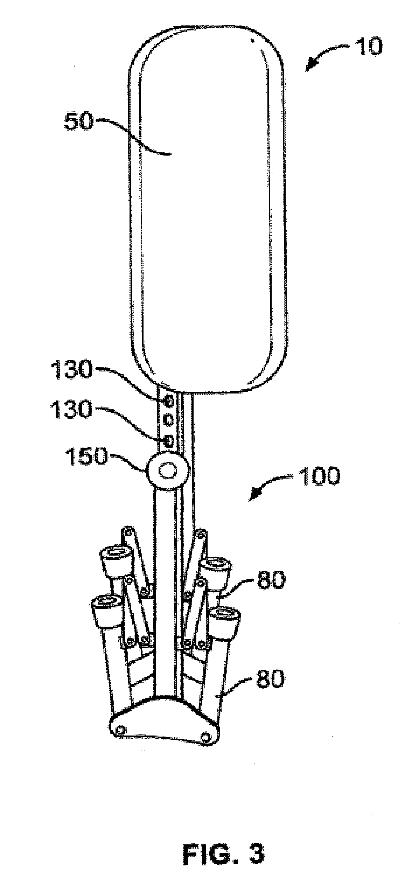

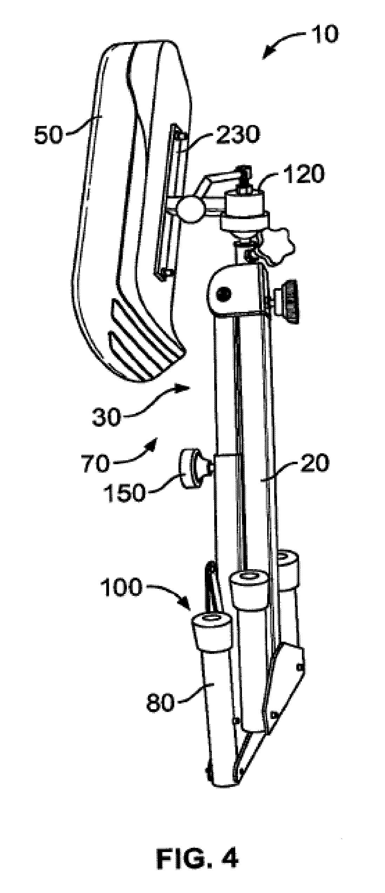

A second support post 30 has a top end 38 and a bottom end 32. The top end 38 of the second support post 30 is pivotally fixed with the top end 28 of the first support post 20 between an extended position 60 and a collapsed position 70 (FIGS. 3 and 4). Preferably the second support post 30 is made from a rigid metallic, steel material.

The bottom end of each support post 20,30 includes two opposing legs 80 each of which are fixed at a proximal end 82 thereof with the bottom end 22,32 of the support post 20,30, respectively. Each leg 80 is pivotal between an extended configuration 90 and a collapsed configuration 100. A collapsible angle brace 110 is fixed between the post 20,30 and a distal end 88 of the leg 80. Each angle brace 110 may be comprised of a first member 210 pivotally attached at a first end 212 thereof to a first end 224 of a second member 220 that is rotationally constrained to between 0 and 180-degrees. Each member 210,210 father includes a second end 218,228, respectively, that is pivotally fixed with either the support post 20,30 or the distal end 88 of one of the legs 80.

Preferably the extended configuration 90 allows the legs 80 to lift each support post 20,30 above the horizontal surface 15 sufficiently to allow a person's foot (not shown) to slide below one of the support posts 20,30 so that a person can adjust the position of the armrest apparatus 10 on the horizontal surface 15 with his foot. Alternately, the extended configuration 90 includes each leg 80 lying substantially flat on the horizontal surface 15 (not shown).

Preferably the bottom end 22,32 of each support post 20,30 is fixed with the two opposing legs 80 at a second pivot mechanism 160. Preferably each leg 80 is made from a rigid metallic, steel material. Alternately, each leg 80 may be made from a strong, rigid plastic or resin material. Each leg 80 may include a plastic cap 200 for covering the distal end 88 thereof.

An armrest support post 40 is telescopingly fixed with the open top end 28 of the first post 20 at a lower end 42 thereof. The armrest support post 40 is fixed with an armrest adjustment mechanism 120 at an upper end 48 thereof. Preferably the armrest support post 40 includes a plurality of adjustment apertures 130 or recesses along one side 43 thereof (FIG. 1), and the second support post 30 is pivotally fixed with the first support post 20 at a first pivot mechanism 140. An adjustment knob 150 traversing the first pivot mechanism 140 and the first support post 20 engages one of the adjustment apertures 130 of the armrest support post 40 to selectively fix the first support post 20 and the armrest support post 40 mutually together. Preferably, the first support post 20 and the armrest support post 40 are each non-circular in cross-section, such as square in cross section as illustrated, so as to prevent mutual rotation thereof.

In one embodiment, the armrest support post 40 terminates at a rotational adjustment mechanism 180 that is fixed with the armrest adjustment mechanism 120. Such a rotational adjustment mechanism 180 includes an adjustment knob 150 traversing the armrest support post 40 to contact a rotational post 190 fixed with the armrest adjustment mechanism 120. As such, the rotational angle of the armrest 50 and the armrest adjustment mechanism 120 is adjusted by loosening the adjustment knob 150, positioning the armrest 50 and the armrest adjustment mechanism 120, and then tightening the adjustment knob 150 to press the rotational post 190 against the armrest support post 140.

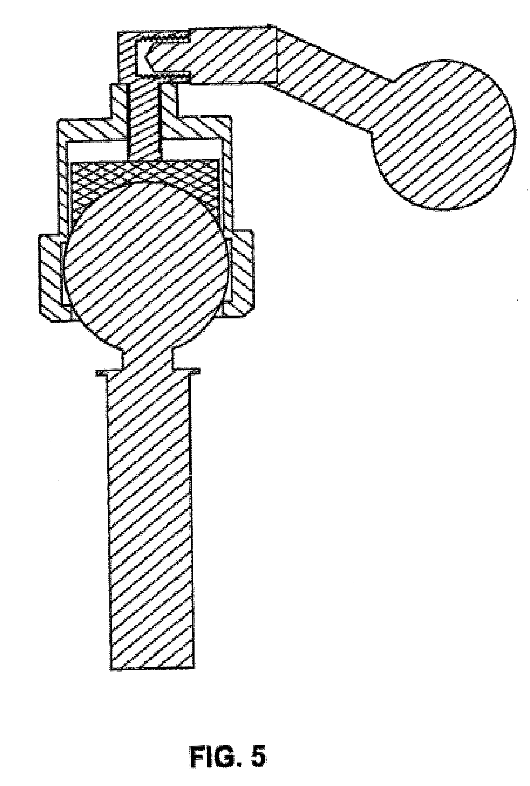

A cushioned armrest 50 is fixed with the armrest adjustment mechanism 120, preferably with a ball joint 170 (FIG. 5) for allowing the armrest 50 to be adjusted angularly with respect to the armrest support post 40, in both tilt and roll directions. The ball joint 170 preferably includes a ball 240 rotationally fixed within a socket 250, and an adjustment lever 270 adapted to move a plunger 260 between a locked position 280 and an unlocked position (not shown), the locked position 280 forcing the plunger 260 into the ball 240 so as to frictionally fix the ball 240 with respect to the socket 250.

The entire armrest apparatus 10 may be rotated on the horizontal surface 15, or the rotational adjustment mechanism 180 may be adjusted, to affect a change in the yaw direction of the armrest 50. In one embodiment, the armrest 50 is fixed with the armrest adjustment mechanism 120 at a rigid base plate 230. The armrest 50 is preferably made from either an open or closed cell pliable foam material.

In use, the first and second support posts 20,30 may be separated into the extended position 60, each leg 80 placed in its extended configuration 90 for supporting the armrest 10 on the horizontal surface 15. The height of the armrest support post 40 may be adjusted within the first support post 20, and the angle of the armrest 50 may be adjusted with the armrest adjustment mechanism 120. The armrest apparatus 10 may be placed in a position suited for supporting the arm or arms of a person (not shown).

Preferably the second support post 30 comprises an upper post 36 and a lower post 34, wherein the upper post 36 includes a plurality of adjustment apertures 130 along one side thereof. An adjustment knob 150 traverses the lower post 34 to engage one of the adjustment apertures 150 of the upper post 36 to selectively fix the lower post 34 and the upper post 36 mutually together at a desired second support post length. Such an arrangement allows the angle of the first support post 20 and armrest support post 40 to be adjusted with respect to the horizontal surface 15.

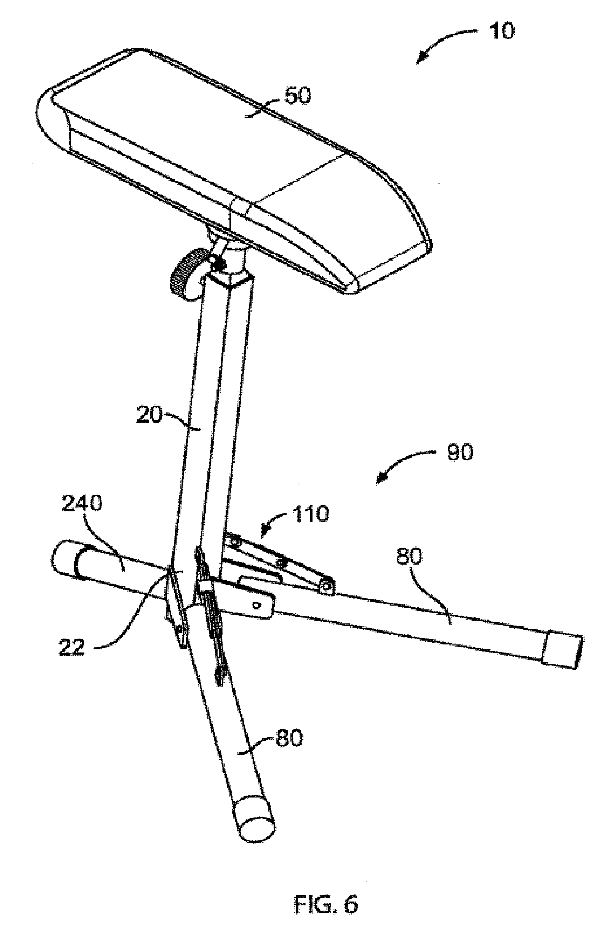

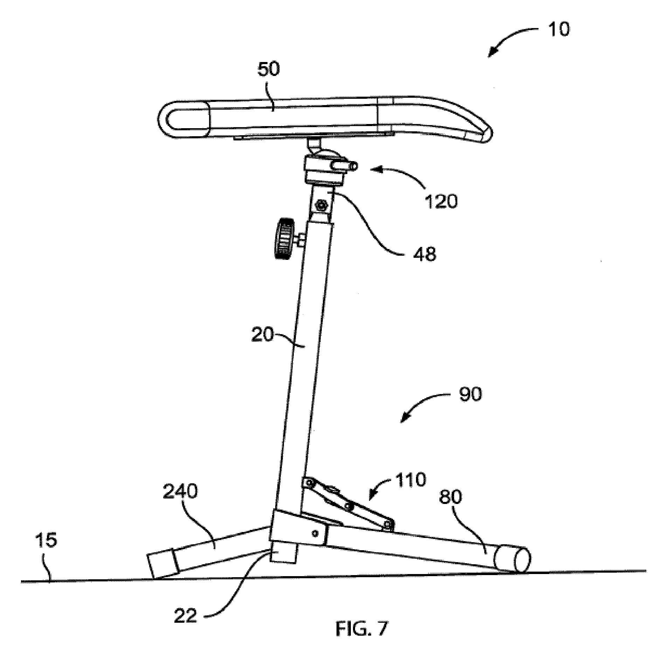

In an alternate embodiment of the invention, illustrated in FIGS. 6 and 7, the armrest apparatus 10 comprises the first support post 20 having the open top end 28 and a bottom end 22, the bottom end 22 including two of the legs 80 each fixed at a proximal end 82 thereof with the bottom end 22 of the support post 20 and pivotal between the extended configuration 90 and the collapsed configuration 100. The collapsible angle brace 110 is fixed between the support post 20 and the distal end of the leg 88, with each leg 80 projecting away from the support post 20 at an angle of less than 180-degrees with respect to the other leg 80. A forward leg 240 extends forwardly away from the bottom end 22 of the support post 20 at an angle of more than 90-degrees with respect to each of the two legs 80. In such an embodiment, the forward leg projecting forwardly from the bottom end of the support post and the two legs projecting away from the support post together support the armrest apparatus on the horizontal surface, precluding the need for the second support post.

While a particular form of the invention has been illustrated and described, it will be apparent that various modifications can be made without departing from the spirit and scope of the invention. Accordingly, it is not intended that the invention be limited, except as by the appended claims.

Particular terminology used when describing certain features or aspects of the invention should not be taken to imply that the terminology is being redefined herein to be restricted to any specific characteristics, features, or aspects of the invention with which that terminology is associated. In general, the terms used in the following claims should not be construed to limit the invention to the specific embodiments disclosed in the specification, unless the above Detailed Description section explicitly defines such terms. Accordingly, the actual scope of the invention encompasses not only the disclosed embodiments, but also all equivalent ways of practicing or implementing the invention.

The above detailed description of the embodiments of the invention is not intended to be exhaustive or to limit the invention to the precise form disclosed above or to the particular field of usage mentioned in this disclosure. While specific embodiments of, and examples for, the invention are described above for illustrative purposes, various equivalent modifications are possible within the scope of the invention, as those skilled in the relevant art will recognize. Also, the teachings of the invention provided herein can be applied to other systems, not necessarily the system described above. The elements and acts of the various embodiments described above can be combined to provide further embodiments.

All of the above patents and applications and other references, including any that may be listed in accompanying filing papers, are incorporated herein by reference. Aspects of the invention can be modified, if necessary, to employ the systems, functions, and concepts of the various references described above to provide yet further embodiments of the invention.

Changes can be made to the invention in light of the above "Detailed Description." While the above description details certain embodiments of the invention and describes the best mode contemplated, no matter how detailed the above appears in text, the invention can be practiced in many ways. Therefore, implementation details may vary considerably while still being encompassed by the invention disclosed herein. As noted above, particular terminology used when describing certain features or aspects of the invention should not be taken to imply that the terminology is being redefined herein to be restricted to any specific characteristics, features, or aspects of the invention with which that terminology is associated.

While certain aspects of the invention are presented below in certain claim forms, the inventor contemplates the various aspects of the invention in any number of claim forms. Accordingly, the inventor reserves the right to add additional claims after filing the application to pursue such additional claim forms for other aspects of the invention.

## Claims

1. An armrest apparatus for resting on a horizontal surface, comprising:
a first support post having an open top end and a bottom end;
a second support post having a top end and a bottom end, the top end of the second support post pivotally fixed with the top end of the first support post between an extended and a collapsed position;
the bottom end of each support post including two opposing legs each fixed at a proximal end thereof with the bottom end of the support post and pivotal between an extended and a collapsed configuration, a collapsible angle brace fixed between the post and a distal end of the leg;
an armrest support post telescopingly fixed within the open top end of the first post at a lower end thereof and fixed with an armrest adjustment mechanism at an upper end thereof; and
a cushioned armrest fixed with the armrest adjustment mechanism, wherein the armrest adjustment mechanism includes a ball joint for allowing the armrest to be adjusted angularly with respect to the armrest support post..

2. The armrest apparatus of claim 1 wherein the armrest support post includes a plurality of adjustment apertures along one side thereof, the second support post pivotally fixed with the first support post at a first pivot mechanism, an adjustment knob traversing the first pivot mechanism and the first support post to engage one of the adjustment apertures of the armrest support post to selectively fix the first support post and the armrest support post mutually together.

3. The armrest apparatus of claim 1 or 2 wherein the second support post comprises an upper post and a lower post, the upper post including a plurality of adjustment apertures along one side thereof, an adjustment knob traversing the lower post to engage one of the adjustment apertures of the upper post to selectively fix the lower post and the upper post mutually together at a desired length.

4. The armrest apparatus of any one of claims 1 to 3 wherein the bottom end of each support post is pivotally fixed with the two opposing legs at a second pivot mechanism.

5. The armrest apparatus of any one of claims 1 to 4 wherein the armrest is formed from a closed-cell foam material.

6. The armrest apparatus of any one of claims 1 to 4 wherein the armrest is formed from an open-cell foam material.

7. The armrest apparatus of any one of the preceding claims wherein each support post is made from a rigid metallic material.

8. The armrest apparatus of any one of the preceding claims wherein each support post is made from a rigid steel material.

9. The armrest apparatus of claim 3 wherein the adjustment knob is made from a rigid steel material.

10. The armrest apparatus of any one of the preceding claims wherein the armrest support post terminates at a rotational adjustment mechanism that is fixed with the armrest adjustment mechanism, the rotational adjustment mechanism including an adjustment knob traversing the armrest support post to contact a rotational post fixed with the armrest adjustment mechanism, whereby the rotational angle of the armrest and armrest adjustment mechanism is adjusted by loosening the adjustment knob, positioning the armrest and armrest adjustment mechanism, and then tightening the adjustment knob to press the rotational post against the armrest support post.

11. The armrest apparatus of any one of the preceding claims wherein each leg is made from a rigid metal material, each leg further including a plastic cap for covering the distal end thereof.

12. The armrest apparatus of any one of the preceding claims wherein each angle brace is comprised of a first member pivotally attached at a first end thereof to a first end of a second member rotationally constrained to between 0 and 180 degrees, each member further including a second end pivotally fixed with either the support post or the distal end of one of the legs.

13. The armrest apparatus of any one of the preceding claims wherein the armrest is fixed with the armrest adjustment mechanism at a rigid base plate.

14. The armrest apparatus of any one of the preceding claims wherein the ball joint includes a ball rotationally fixed within a socket, and an adjustment lever adapted to move a plunger between a locked position and an unlocked position, the locked position forcing the plunger into the ball so as to frictionally fix the ball with respect to the socket.
